# FASCICULE DE BREVET EUROPEEN

(11) **EP 1 216 018 B1**
(45) Date de publication et mention de la délivrance du brevet: **19.07.2006**
(21) Numéro de dépôt: 99946237.7
(22) Date de dépôt: 27.09.1999
(51) Int. Cl.: A61Q 5/06, A61K 8/58

(54) **COMPOSITION COSMETIQUE A BASE DE COMPOSES ORGANIQUES DU SILICIUM PARTIELLEMENT NEUTRALISES**
KOSMETISCHE ZUSAMMENSETZUNG AUF BASIS VON PARTIELL NEUTRALISIERTEN ORGANO-SILIZIUM VERBINDUNGEN
COSMETIC COMPOSITIONS BASED ON PARTLY NEUTRALISED ORGANIC SILICON COMPOUNDS

(43) Date de publication de la demande: 26.06.2002
(73) Titulaire: L'ORÉAL, 75008 Paris (FR)
(72) Inventeur: SAMAIN, Henri, 91570 Bièvres (FR); ROLLAT, Isabelle, 92100 Boulogne-Billancourt (FR); JEANNE ROSE, Valérie, 75019 Paris (FR); SANCHEZ, Clément, 91190 Gif-sur-Yvette (FR)
(74) Mandataire: Casalonga, Axel
(86) Numéro de dépôt international: PCT/FR1999/002289
(87) Numéro de publication internationale: WO 2001/022925

(56) Documents cités:
- EP-A- 0 279 623
- EP-A- 0 877 027
- WO-A-79/00454
- WO-A-89/04163
- CH-A- 535 579
- US-A- 5 750 092
- BASE DE DONN¹ES "CHEMICAL ABSTRACTS" (SERVEUR STN, Karlsruhe, DE); abrégé 111:102 511; & JP 63 307 811 A (KANEBO, Ltd) 15 D¹CEMBRE 1988 XP002107176

## Description

La présente invention concerne d'une manière générale des compositions cosmétiques aqueuses; en particulier pour le traitement des cheveux, comportant des composés organiques du silicium solubles dans l'eau, peu ou pas polymérisés.

Il est habituel d'utiliser des composés organiques tels que des polymères pour réaliser des compositions cosmétiques pour le traitement des cheveux. Par exemple, on utilise des polymères donnant au sèchage des matériaux solides pour fixer dans une forme la coiffure. De tels matériaux sont également utilisés pour donner des effets de maintien de la forme. On utilise aussi des composés polymériques, tels que des polysiloxanes, pour donner des effets de soin aux cheveux, particulièrement les cheveux abîmés ou difficiles à démêler. Les compositions cosmétiques contenant ces polymères sont déposées sur les cheveux qu'on laisse sécher ou que l'on rince avant de passer au séchage.

L'utilisation de composés polymériques présente plusieurs inconvénients.

Le premier inconvénient réside dans le fait que, lorsque les polymères sont utilisés dans des compositions au-delà d'une certaine concentration, les compositions obtenues s'appliquent difficilement du fait de l'accroissement de la viscosité de la composition. Cette difficulté d'application des compositions entraîne des surcharges en certains endroits de la chevelure et donc des défauts cosmétiques et implique également que certaines parties de la chevelure reçoivent moins de compositions, ce qui, au final, induit sur ces parties un moindre effet.

Le second inconvénient réside dans le fait que ces compositions sont parfois difficiles à mettre en oeuvre. En effet, les composés polymériques à faible solubilité dans l'eau, exigent l'utilisation de solvant organique ou de mélange de solvants organiques. L'emploi de solvant organique entraîne plusieurs problèmes, comme des problèmes d'environnement et d'effet sur la cosméticité des cheveux.

Pour remédier à ces inconvénients, on s'est donc tourné vers l'utilisation de composés polymériques rendus partiellement solubles dans l'eau. Ainsi, certains composés polymériques peuvent être utilisés dans l'eau sans ajout d'un quelconque co-solvant. Dans ce cas, la limitation réside dans le fait que ces composés polymériques sont éliminés partiellement, voire totalement, par rinçage des cheveux. Par conséquent, dans ce cas, l'effet dû aux composés polymériques est très limité après rinçage. Au final, cela limite l'effet des traitements rincés (shampooing, après shampooing), mais réduit aussi l'intérêt de telles compositions utilisées en mode non rincé (laques, mousses, lotions de mise en plis) dans la mesure où l'utilisateur perd l'effet acquis par le traitement lorsqu'il se lave les cheveux.

Des efforts ont donc été réalisés pour trouver des composés pour la formulation de compositions cosmétiques qui soient utilisables dans l'eau et qui présentent une rémanence de leur effet lorsque les cheveux sont rincés.

Ainsi, le brevet des Etats-Unis n° 4 344 763 (GILLETTE) décrit des compositions cosmétiques comportant un monomère organosiloxane tel qu'un aminoalkylalcoxysilane et un titanate organique en solution dans un alcool.

Plus précisément, ce brevet décrit un procédé de mise en forme des cheveux qui consiste à les humidifier avec de l'eau puis à appliquer une solution contenant dans de l'isopropanol de 0,5 à 15% en poids d'un aminoalkylalcoxysilane et de 0,005 à 1,5% en poids d'un titanate organique et à mettre ensuite les cheveux dans la forme souhaitée.

Selon ce procédé, il est particulièrement recommandé de maintenir la solution d'isopropanol à l'abri de toute humidité.

Il a également été décrit dans le brevet EP-113 992, un procédé pour simultanément fixer et conditionner les cheveux à l'aide d'une composition, stable en l'absence d'humidité, contenant (A) un oligomère de siloxane ayant au moins une liaison azote-hydrogène, et (B) un additif anhydre, facilement hydrolysable, choisis parmi les titanates, zirconates, vanadates, germanates, et leurs mélanges.

Le solvant de la composition est un hydrocarbure aliphatique ou un halogénohydrocarbure aliphatique, de préférence le 1,1,1-trichloroéthane.

Après application de la composition sur les cheveux, ceux-ci sont placés en atmosphère humide afin de provoquer la réticulation de l'oligomère de siloxane et de l'additif anhydre facilement hydrolysable.

Il existe donc un besoin d'une composition cosmétique stable, en particulier pour le traitement des cheveux, qui soit essentiellement aqueuse et qui permette d'obtenir un effet cosmétique suffisant, en particulier pour les cheveux en mode rincé ou non rincé.

La présente invention a donc pour objet des compositions cosmétiques aqueuses, stables, en particulier des compositions cosmétiques pour le traitement et le soin des cheveux, qui remédient aux inconvénients de l'art antérieur.

Plus précisément, la présente invention a pour objet des compositions cosmétiques aqueuses, stables, pour le traitement et le soin des cheveux, conférant aux cheveux un effet coiffant de longue durée et un toucher agréable.

Le Demandeur a remarqué, de façon surprenante, qu'il était possible de formuler des compositions cosmétiques ne nécessitant pas l'utilisation de solvant organique et qui présentaient un effet cosmétique efficace, résistant au rinçage, sans risque de problèmes de cheveux chargés en cas de superposition, en utilisant dans ces compositions des composés organiques du silicium peu ou pas polymérisés, solubles dans l'eau, comportant au moins une fonction chimique basique et partiellement neutralisée.

Certains composés organiques du silicium sont connus de US 5 750 092. Ce document décrit l'utilisation d'alcoxysilanes comportant une fonction éthylènediamine, associés à de la dihydroxyacétone et à un modificateur de couleur, pour obtenir un bronzage artificiel de la peau.

Cependant, on a observé que l'application de compositions telles que décrites ci-dessous permet d'obtenir des effets cosmétiques marqués, sans problème en cas de superposition, dont les effets résistent bien au rinçage et au lavage.

Selon l'invention, les compositions cosmétiques, en particulier pour le traitement des cheveux, comprennent, dans un milieu aqueux cosmétiquement acceptable, au moins 0,02% en poids par rapport au poids total de la composition, d'un ou plusieurs composés organiques du silicium solubles dans l'eau, peu ou pas polymérisés, choisis parmi les organosilanes comportant un atome de silicium tels que décrits ci-dessous, et les organosiloxanes comportant deux ou trois atomes de silicium, les composés organiques du silicium comportant en outre au moins une fonction chimique basique et au moins deux groupes hydrolysables ou hydroxyles par molécule, caractérisée en ce qu'elle comprend une quantité d'un agent de neutralisation telle que les composés organiques du silicium, peu ou pas polymérisés, sont neutralisés à raison de 1/1000 à 99/100, de préférence de 0,2/100 à 70/100.

Les composés organiques du silicium selon l'invention sont susceptibles de former, en milieux aqueux, un composé non hybride, après condensation sur eux-mêmes et évaporation du support. On entend par composé non hybride, un composé chimiquement homogène quant au silicium, c'est-à-dire qu'il ne renferme pas d'autres espèces métalliques ou organométalliques supplémentaires.

Les composés organiques du silicium, peu ou pas polymérisés, utiles dans les compositions de la présente invention sont choisis parmi les organosilanes solubles dans l'eau, comprenant un atome de silicium tels que décrits ci-après et les organosiloxanes solubles dans l'eau, comportant deux ou trois atomes de silicium, de préférence deux atomes de silicium. Ils doivent en outre comporter au moins une fonction chimique basique, et de préférence une seule fonction chimique basique. La fonction chimique basique peut être toute fonction conférant un caractère basique au composé de silicium sans nuire à sa solubilité dans l'eau et est de préférence une fonction amine telle qu'une fonction amine primaire, secondaire ou tertiaire..La fonction chimique basique des composés du silicium selon l'invention, peut comporter éventuellement d'autres fonctions, telles que, par exemple, une autre fonction amine, une fonction acide ou une fonction halogène.

Les composés organiques du silicium utiles dans les compositions de la présente invention, comportent en outre au moins deux groupes hydrolysables ou hydroxyles par atome de silicium. Les groupes hydrolysables sont de préférence des groupes alcoxy, aryloxy ou halogène. Ils peuvent également, éventuellement, comporter d'autres fonctions chimiques telles que dés fonctions acides ou amines.

Les organosilanes utilisés dans l'invention répondent à la formule : 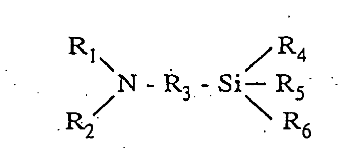 dans laquelle :
R₄ représente un halogène, un groupe OR' ou R'₁ ;
R₅ représente un halogène, un groupe OR" ou R'₂ ;
R₆ représente un halogène, un groupe OR''' ou R'₃ ;
et R₁, R₂, R₃, R', R", R''', R'₁, R'₂, R'₃ représentent, indépendamment les uns des autres, un groupe hydrocarboné saturé ou insaturé, linéaire ou ramifié, portant éventuellement des groupes chimiques supplémentaires acides, R₁, R₂, R', R" et R''' pouvant en outre désigner l'hydrogène, et
deux au moins des groupes R₄, R₅ et R₆ étant différents des groupes R'₁, R'₂ et R'₃.

De préférence, R₁, R₂, R', R" et R"', R'₁, R'₂ et R'₃ représentent un groupe alkyle de C₁ à C₁₂, un groupe aryle de C₆ à C₁₄, un groupe alkyle de C₁ à C₈-aryle de C₆ à C₁₄, et un groupe aryle de C₆ à C₁₄-alkyle C₁ à C₈; et R₃ est de préférence un groupe alkyle dé C₁ à C₁₂, aryle de C₆ à C₁₄, alkyle de C₁ à C₈-aryle de C₆ à C₁₄ et aryle de C₆ à C₁₄-alkyle de C₁ à C₈.

Les organosiloxanes préférés dans les compositions de la présente invention peuvent être représentés par la formule : 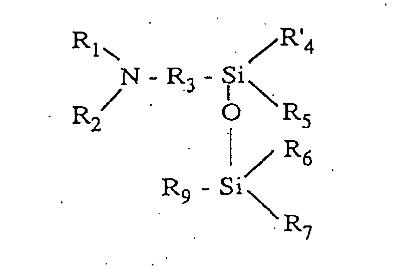 dans laquelle :
R₁, R₂, R₃, R₅ et R₆ sont définis comme précédemment;
R'₄ représente un halogène ou un groupe OR₁₁;
R₇ représente un halogène, un groupe OR₁₀ ou R"₁;
R₉ représente un halogène, un groupe OR₈, R"₂ ou R₃NR₁R₂;
R"₁, R"₂, R₈, R₁₀ et R₁₁ représentent un groupe hydrocarboné, saturé ou insaturé, linéaire ou ramifié, portant éventuellement des groupes chimiques supplémentaires tels que des groupes solubilisants basiques;
R₁₁, R₁₀ et R₈ pouvant en outre désigner l'hydrogène.

De préférence R"₁, R"₂, R₈ ou R₁₀ et R₁₁ représentent un groupe alkyle de C₁ à C₁₂, un groupe aryle de C₆ à C₁₄, un groupe alkyle de C₁ à C₈₋aryle de C₆ à C₁₄, et un groupe aryle de C₆ à C₁₄-alkyle de C₁ à C₈.

L'un au moins des groupes R₆, R₇ et R₉ désigne un halogène ou un groupe OR"', OR₁₀ ou OR₈.

De préférence, l'halogène est le chlore.

Un aspect important des compositions de l'invention est que les composés organiques de silicium, peu ou pas polymérisés, sont partiellement neutralisés au moyen d'un agent de neutralisation ou régulateur de pH, de telle sorte que la neutralisation atteigne 1/1000 à 99/100 et mieux de 0,2/100 à 70/100. De préférence encore, la neutralisation est de 0,2/100 à 60/100.

Les agents régulateurs de pH sont choisis parmi l'acide chlorhydrique, l'acide nitrique et les acides organiques mono, di ou tri-carboxyliques.

Cette neutralisation partielle des composés organiques du silicium peu ou pas polymérisés des compositions de l'invention revêt un aspect important pour l'obtention des propriétés voulues pour les compositions.

Un autre aspect important des compositions selon l'invention est que les composés organiques du silicium, les agents régulateurs de pH ainsi que les autres constituants de la composition, sont choisis de façon à ce que cette composition contienne des quantités importantes des composés organiques du silicium peu ou pas polymérisés, c'est-à-dire comportant un, deux ou trois atomes de silicium. Ainsi, il est nécessaire que la composition contienne, par rapport au poids total de la composition, au moins 0,02% de composés organiques du silicium peu ou pas polymérisés et de préférence au moins 0,5% et pouvant aller jusqu'à 50% en poids.

Le taux des composés organiques du silicium peu ou pas polymérisés, selon l'invention, est déterminé par des méthodes habituelles d'analyse telles que la spectroscopie RMN du silicium 29 et du proton, et par chromatographie.

Les compositions selon l'invention sont des compositions aqueuses. Toutefois, il est possible, pour la mise en oeuvre d'adjuvants par exemple, d'ajouter un co-solvant tel qu'un alcool ou une cétone, par exemple l'alcool éthylique ou l'acétone.

De façon connue, toutes les compositions de l'invention peuvent contenir les adjuvants habituels dans le domaine cosmétique, tels que des huiles, cires ou autres corps gras usuels; des gélifiants et/ou épaississants classiques; des émulsionnants; des agents hydratants; des émollients, des filtres solaires; des actifs hydrophiles ou lipophiles comme des céramides; des agents anti-radicaux libres; des tensio-actifs; des polymères; des protéines; des bactéricides; des séquestrants; des antipelliculaires; des anti-oxydants; des conservateurs; des parfums; des charges; des matières colorantes.

Les quantités de ces différents adjuvants sont celles classiquement utilisées dans le domaine considéré.

Bien entendu, l'homme de l'art veillera à choisir le ou les éventuels composés ajoutés à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention sont utilisables en mode rincé ou non rincé.

Les compositions selon l'invention peuvent se présenter sous toutes les formes appropriées pour une application topique, notamment sous forme de solutions du type lotions ou sérum; sous forme de gels aqueux; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses.

Ces compositions sont préparées selon les méthodes usuelles.

Les compositions selon l'invention sont de préférence utilisées comme produits capillaires, notamment pour le maintien de la coiffure ou de la mise en forme des cheveux. Elles peuvent en outre apporter de la coloration temporaire aux cheveux, bien assurer la protection des cheveux contre les effets des radiations UV, tout en apportant des propriétés de maintien ou de fixation des cheveux.

Les compositions capillaires, selon l'invention, sont de préférence des produits de coiffage tels que des gels, des lotions de mises en pli, des lotions pour le brushing, des compositions de fixation et de coiffage telles que des laques ou spray.

Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsqu'on souhaite obtenir un spray, une mousse pour la fixation ou le traitement des cheveux.

La présente invention a également pour objet l'utilisation de la composition selon l'invention dans un procédé de traitement des cheveux, en vue de leur maintien et/ou coloration.

Selon un mode de réalisation de ce procédé, on applique la composition sur les cheveux rincés ou non, de préférence sous la forme d'un spray, soit à l'aide d'un flacon pompe, soit à l'aide d'un aérosol.

Après pulvérisation sur l'ensemble de la chevelure, on laisse agir et sécher la composition.

Les cheveux peuvent être rincés après application de la composition.

Les cheveux peuvent être mis dans la forme souhaitée, soit avant l'application, soit immédiatement après.

Le temps de séchage peut être variable et est fonction de la nature de la composition.

Les cheveux, après peignage, présentent une qualité de toucher très agréable.

L'invention est illustrée par les exemples suivants :

### EXEMPLE 1

On a réalisé les quatre formulations suivantes :

| Compositions | Composé du silicium peu ou pas polymérisé, soluble dans l'eau | Agent de neutralisation | Eau |
|---|---|---|---|
| | Aminopropyltriéthoxysilane (g pour 100 g de composition) | Acide chlorhydrique (quantité de neutralisation (normalité) par rapport à la quantité de silane soluble) | |
| 1 | 12 g | 0 | qsp 100 g |
| 2 | 12 g | 0,5 | qsp 100 g |
| 3 | 12 g | 0,25 | qsp 100 g |
| 4 | 12 g | 0,75 | qsq 100 g |

L'analyse RMN du silicium 29 montre que ces quatre compositions contiennent environ 10 g de composés organiques du silicium ayant un, deux ou trois atomes de silicium pour 100 g de composition (pics RMN présents à un déplacement chimique allant de -30 à -75 ppm (le tétraméthylsilane servant de référence)).

Les quatre compositions sont utilisées dans les applications suivantes :

### Application 1 : Application non rincée. Obtention d'un effet coiffant.

Les compositions sont introduites dans un flacon aérosol à raison de 65 g. On aménage le flacon aérosol d'une valve 51 P et d'un diffuseur C02 045. On ajoute dans chaque flacon 35 g de diméthyl éther.

On prépare deux mèches de 5 g de cheveux naturels. Les cheveux sont maintenus aux racines par une attache et sont disposés en triangle.

Les compositions 1, 2 et 3 sont propulsées pendant 5 secondes par face sur les mèches. Après séchage (15 minutes), on fait évaluer par un panel de 8 testeurs entraînés, l'effet coiffant sur une échelle de 0 à 50; 0 correspond à un effet coiffant nul et 50 à un effet coiffant très fort.

On procède à un démêlage et on fait évaluer par un panel de 8 testeurs entraînés, les qualités de toucher des mèches ainsi traitées.

La qualité de toucher est notée sur une échelle de 0 à 50; 0 correspond à une qualité de toucher très médiocre et 50 à une qualité de toucher très agréable.

On totalise pour chaque composition les notes des 8 testeurs, puis on fait la moyenne.

On obtient les résultats suivants :

| Compositions | Moyenne des notes "effet coiffant" | Moyenne des notes "qualité de toucher" "après démêlage" |
|---|---|---|
| 1 | 10 | 15 |
| 2 | 30 | 20 |
| 3 | 30 | 15 |

Les résultats montrent qu'avec les compositions partiellement neutralisées, on obtient un meilleur effet coiffant et une qualité de toucher après démêlage au moins égale à ceux d'une composition non neutralisée.

### Application 2 : Application non rincée. Obtention d'un effet coiffant.

On prépare deux mèches de 5 g de cheveux naturels. Les cheveux sont maintenus aux racines et laissés libres sur le reste de la longueur.

Les compositions sont mises en contact avec les mèches pendant 2 minutes (les mèches sont immergées dans 10 ml de la solution à tester). Elles sont ensuite laissées à sécher pendant 24 heures.

Après séchage, les cheveux sont collés les uns aux autres dans tous les cas.

On procède à un démêlage et on fait évaluer par un panel de 8 testeurs entraînés les qualités de toucher des mèches ainsi traitées.

La qualité de toucher est notée sur une échelle de 0 à 50; 0 correspond à une qualité de toucher très médiocre et 50 à une qualité de toucher très agréable.

On totalise pour chaque composition les notes des 8 testeurs, puis on fait la moyenne.

Le tableau résume les résultats :

| Compositions | Moyenne des notes "qualité de toucher après démêlage" |
|---|---|
| 1 | 10 |
| 2 | 25 |
| 3 | 25 |
| 4 | 15 |

Les résultats montrent qu'avec les compositions partiellement neutralisées selon l'invention, on obtient une meilleure qualité de toucher après démêlage, en application non rincée, par rapport à une composition non neutralisée.

## Revendications

1. Composition cosmétique comprenant, dans un milieu aqueux cosmétiquement acceptable, au moins 0,02% en poids par rapport au poids total de la composition d'un ou plusieurs composés organiques du silicium soluble dans l'eau, peu ou pas polymérisés, choisis parmi les silanes comprenant un atome de silicium de formule : 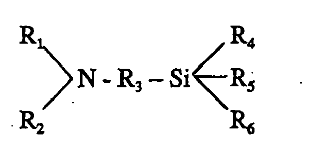 dans laquelle :
R₄ représente un halogène ou un groupe OR' ou R'₁;
R₅ représente un halogène ou un groupe OR" ou R'₂;
R₆ représente un halogène ou un groupe OR"' ou R'₃;
R₁, R₂, R₃, R', R", R"', R'₁, R'₂ et R'₃ représentent, indépendamment les uns des autres, un groupe hydrocarboné saturé ou insaturé, linéaire ou ramifié, portant éventuellement des groupes chimiques supplémentaires acides,
R₁, R₂, R', R" et R"' pouvant en outre désigner l'hydrogène, deux au moins des groupes R₄, R₅ et R₆ étant différents des groupes R'₁, R'₂ et R'₃,
et les siloxanes comprenant deux ou trois atomes de silicium, ces composés organiques du silicium comportant en outre au moins une fonction chimique basique et au moins deux groupes hydroxyles ou hydrolysables par molécule, composition **caractérisée en ce qu'**elle comprend une quantité d'un agent de neutralisation telle que les composés organiques du silicium, peu ou pas polymérisés, sont neutralisés à raison de 1/1000 à 99/100, dé préférence de 0,2/100 à 70/100, ledit agent de neutralisation étant choisi parmi l'acide chlorhydrique, l'acide nitrique et les acides organiques mono-, di- et tricarboxyliques.

2. Composition cosmétique selon la revendication 1, **caractérisée en ce que** les composés organiques du silicium, peu ou pas polymérisés, solubles dans l'eau, représentent au moins 0,5% et jusqu'à 50% en poids de la composition.

3. Composition selon la revendication 1 ou 2, **caractérisée en ce que** la fonction chimique basique des composés organiques du silicium est choisie parmi les amines primaires, secondaires ou tertiaires.

4. Composition selon l'une quelconque des revendicatioris 1 à 3, **caractérisée en ce que** les groupes hydrolysables sont choisis parmi les groupes alcoxy, aryloxy et halogène.

5. Composition cosmétique selon l'une quelconque des revendications précédentes, **caractérisée en ce que** les siloxanes sont représentés par la formule : 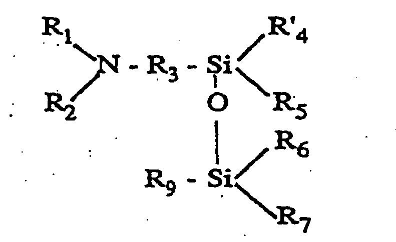 dans laquelle :
R₁, R₂, R₃, R₅ et R₆ sont définis comme précédemment;
R'₄ représente un halogène ou un groupe OR₁₁;
R₇ représente un halogène, un groupe OR₁₀ ou R"₁;
R₉ représente un halogène, un groupe OR₈, R"₂ ou R₃NR₁R₂;
R"₁, R"₂, R₈, R₁₀ et R₁₁ représentent un groupe hydrocarboné, saturé ou insaturé, linéaire ou ramifié, portant éventuellement des groupes chimiques supplémentaires, les groupes R₁₁, R₁₀ et R₈ pouvant en outre désigner l'hydrogène; l'un au moins des groupes R₆, R₇ et R₉ désignant un halogène, un groupe OR"', OR₁₀ ou OR₈.

6. Composition cosmétique selon la revendication 5, **caractérisée en ce que** les groupes R₁, R₂, R', R'₁, R'₂, R'₃, R", R"', R"₁, R"₂, R₈, R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

7. Composition selon l'une quelconque des revendications précédentes, **caractérisée par le fait qu'**il s'agit d'un produit capillaire.

8. Composition selon la revendication 7, **caractérisée par le fait qu'**il s'agit d'un produit capillaire pour le maintien de la coiffure ou la mise en forme des cheveux.

## Claims

1. Cosmetic composition comprising, in a cosmetically acceptable aqueous medium, at least 0.02% by weight, relative to the total weight of the composition, of one or more unpolymerized or substantially polymerized, water-soluble organosilicon compounds chosen from silanes comprising one silicon atom of formula: 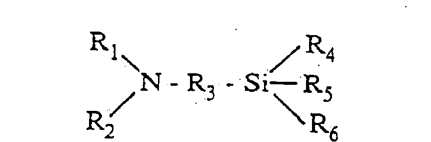 in which:
R₄ represents a halogen or a group OR' or R'₁;
R₅ represents a halogen or a group OR" or R'₂;
R₆ represents a halogen or a group OR"' or R'₃;
R_{1'} R₂, R₃, R', R", R"', R'₁, R'₂ and R'₃ represent, independently of each other, a saturated or unsaturated, linear or branched hydrocarbon group optionally bearing acidic additional chemical groups, R₁, R₂, R', R" and R"' also possibly denoting hydrogen, at least two of the groups R₄, R₅ and R₆ being other than groups R'₁, R'₂ and R'₃, and siloxanes comprising two or three silicon atoms, these organosilicon compounds also comprising at least one basic chemical function and at least two hydrolysable or hydroxyl groups per molecule, **characterized in that** it comprises an amount of a neutralizing agent such that the unpolymerized or substantially polymerized organosilicon compounds are neutralized to a proportion of from 1/1000 to 99/100 and preferably from 0.2/100 to 70/100, said neutralizing agent being chosen from hydrochloric acid, nitric acid and mono-, di- and tricarboxylic organic acids.

2. Cosmetic composition according to Claim 1, **characterized in that** the water-soluble, unpolymerized or substiantially polymerized organosilicon compounds represent at least 0.5% and up to 50% by weight of the composition.

3. Composition according to Claim 1 or 2, **characterized in that** the basic chemical function of the organosilicon compounds is chosen from primary, secondary and tertiary amines.

4. Composition according to any one of Claims 1 to 3, **characterized in that** the hydrolysable groups are chosen from alkoxy, aryloxy and halogen groups.

5. Cosmetic composition according to any one of the preceding claims, **characterized in that** the siloxanes are represented by the formula: 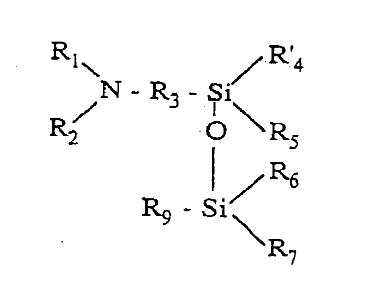 in which:
R₁, R₂, R₃, R₅ and R₆ are defined as above;
R'₄ represents a halogen or a group OR₁₁;
R₇ represents a halogen or a group OR₁₀ or R"₁;
R₉ represents a halogen or a group OR₈, R"₂ or R₃NR₁R₂;
R"₁, R"₂, R₈, R₁₀ and R₁₁ represent a saturated or unsaturated, linear or branched hydrocarbon group optionally bearing additional chemical groups, the groups R₁₁, R₁₀ and R₈ also possibly denoting hydrogen; at least one of the groups R₆, R₇ and R₉ denoting a halogen or a group OR"', OR₁₀ or OR₈.

6. Cosmetic composition according to Claim 5, **characterized in that** the groups R₁, R₂, R', R'₁, R'₂, R'₃, R", R"', R"₁, R"₂, R₈, R₁₀ and R₁₁ are chosen from C₁ to C₁₂ alkyl radicals, C₆ to C₁₄ aryl radicals, (C₁ to C₈)alkyl(C₆ to C₁₄)aryl radicals and (C₆ to C₁₄)aryl(C₁ to C₈)alkyl radicals.

7. Composition according to any one of the preceding claims, **characterized in that** it is a hair product.

8. Composition according to Claim 7, **characterized in that** it is a hair product for holding the hairstyle or for shaping the hair.

## Patentansprüche

1. Kosmetische Zusammensetzung, die in einem kosmetisch akzeptablen, wässrigen Medium, bezogen auf das Gesamtgewicht der Zusammensetzung, mindestens 0,02 Gew.-% einer oder mehrerer, in Wasser löslicher, wenig oder gar nicht polymerisierter Silicium-organischer Verbindungen enthält, die unter den Silanen der folgenden Formel, die ein Siliciumatom enthalten: 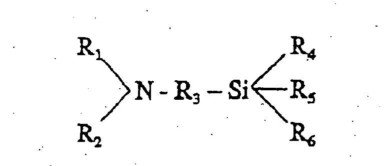 worin bedeuten:
R₄ ein Halogen oder OR' oder R'₁;
R₅ ein Halogen oder OR" oder R'₂;
R₆ ein Halogen oder OR'" oder R'₃;
die Gruppen R₁, R₂, R₃, R', R", R'", R'₁, R'₂ und R'₃ unabhängig voneinander eine gesättigte oder ungesättigte, geradkettige oder verzweigte Kohlenwasserstoffgruppe, die gegebenenfalls zusätzliche saure chemische Gruppen enthalten kann,
wobei R₁, R₂, R', R" und R'" ferner Wasserstoff bedeuten können und mindestens zwei der Gruppen R₄, R₅ und R₆ von den Gruppen R'₁, R'₂ und R'₃ verschieden sind,
und den Siloxanen ausgewählt sind, die zwei oder drei Siliciumatome enthalten, wobei diese Silicium-organischen Verbindungen ferner mindestens eine basische chemische Funktion und mindestens zwei Hydroxygruppen oder hydrolysierbare Gruppen pro Molekül aufweisen, **dadurch gekennzeichnet, dass** sie einen solchen Mengenanteil eines Neutralisationsmittels enthält, das die wenig oder gar nicht polymerisierten Silicium-organischen Verbindungen in einem Anteil von 1 / 1 000 bis 99/ 100 und vorzugsweise 0,2/100 bis 70/100 neutralisiert werden, wobei das Neutralisationsmittel unter Salzsäure, Salpetersäure und organischen Mono-, Di- und Tricarbonsäuren ausgewählt ist.

2. Kosmetische Zusammensetzung nach Anspruch 1, **dadurch gekennzeichnet, dass** die gar nicht oder wenig polymerisierten, in Wasser löslichen Silicium-organischen Verbindungen mindestens 0,5 % und bis zu 50 Gew.-% der Zusammensetzung ausmachen.

3. Zusammensetzung nach Anspruch 1 oder 2, **dadurch gekennzeichnet, dass** die basische chemische Funktion der Silicium-organischen Verbindungen unter den primären, sekundären oder tertiären Aminen ausgewählt ist.

4. Zusammensetzung nach einem der Ansprüche 1 bis 3, **dadurch gekennzeichnet, dass** die hydrolysierbaren Gruppen unter den Gruppen Alkoxy, Aryloxy und Halogen ausgewählt sind.

5. Kosmetische Zusammensetzung nach einem der vorhergehenden Ansprüche, **dadurch gekennzeichnet, dass** die Siloxane durch die folgende Formel dargestellt werden können: 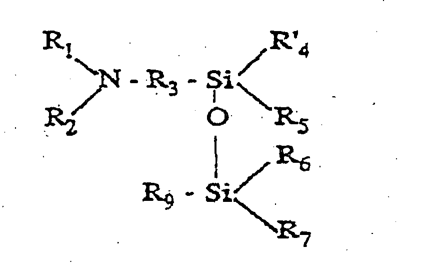 worin bedeuten:
die Gruppen R₁, R₂, R₃, R₅ und R₆ die oben angegebenen Definitionen;
R'₄ ein Halogen oder eine Gruppe OR₁₁;
R₇ ein Halogen, eine Gruppe OR₁₀ oder R"₁;
R₉ ein Halogen, eine Gruppe OR₈, R"₂ oder R₃NR₁R₂;
wobei R"₁, R"₂, R₈, R₁₀ und R₁₁ eine gesättigte oder ungesättigte, geradkettige oder verzweigte Kohlenwasserstoffgruppe bedeuten, die gegebenenfalls zusätzliche chemische Gruppen aufweisen kann, wobei die Gruppen R₁₁, R₁₀ und R₈ ferner Wasserstoff bedeuten können; wobei mindestens eine der Gruppen R₆, R₇ und R₉ ein Halogen, eine Gruppe OR"', OR₁₀ oder OR₈ bedeutet.

6. Kosmetische Zusammensetzung nach Anspruch 5, **dadurch gekennzeichnet, dass** die Gruppen R₁, R₂, R', R'₁, R'₂, R'₃, R", R''', R"₁, R"₂, R₈, R₁₀ und R₁₁ unter den Gruppen C₁₋₁₂-Alkyl, C₆₋₁₄₋Aryl, C₁₋₈-Alkyl-C₆₋₁₄-aryl und C₆₋₁₄-Aryl-C₁₋₈-alkyl ausgewählt sind.

7. Zusammensetzung nach einem der vorhergehenden Ansprüche, **dadurch gekennzeichnet, dass** es sich um ein Produkt für die Haarbehandlung handelt.

8. Zusammensetzung nach Anspruch 7, **dadurch gekennzeichnet, dass** es sich um ein haarkosmetisches Produkt für die Festigung oder die Formgebung der Frisur handelt.
